# EUROPEAN PATENT SPECIFICATION

(11) **EP 3 206 792 B1**
(45) Date of publication and mention of the grant of the patent: **27.05.2020**
(21) Application number: 15778976.9
(22) Date of filing: 13.10.2015
(51) Int. Cl.: B01L 3/00

(54) **MICROFLUIDIC DEVICE WITH ONE MICROCHANNEL FOR MULTIPLE DETECTION**
MIKROFLUIDISCHE VORRICHTUNG MIT EINEM MIKROKANAL FÜR MEHRFACHE DETEKTION
DISPOSITIF MICROFLUIDIQUE AVEC UN MICROCANAL DE DÉTECTION MULTIPLE

(30) Priority: 13.10.2014 EP 14306617
(43) Date of publication of application: 23.08.2017
(73) Proprietor: Paris Sciences et Lettres - Quartier Latin, 75006 Paris (FR); Institut National de la Santé et de la Recherche Médicale (INSERM), 75013 Paris (FR); Université Paris Descartes, 75006 Paris (FR); Centre National de la Recherche Scientifique (CNRS), 75016 Paris (FR)
(72) Inventor: VARENNE, Anne, F-75011 Paris (FR); BEDIOUI, Fethi, F-75010 Paris (FR); GRIVEAU, Sophie, F-91300 Massy (FR); D'ORLYE, Fanny, F-75013 Paris (FR)
(74) Representative: Regimbeau
(86) International application number: PCT/EP2015/073723
(87) International publication number: WO 2016/059080

(56) References cited:
- WO-A1-2010/019969
- WO-A2-2008/034102
- US-A1- 2005 064 581
- SUN XUE-LONG ET AL: "Carbohydrate and protein immobilization onto solid surfaces by sequential Diels-Alder and azide-alkyne cycloadditions", BIOCONJUGATE CHEMISTRY, ACS, WASHINGTON, DC, US, vol. 17, no. 1, 18 January 2006 (2006-01-18), pages 52-57, XP002588543, ISSN: 1043-1802, DOI: 10.1021/BC0502311 [retrieved on 2005-12-21]

### SEQUENCE LISTING

<110> PARIS SCIENCES ET LETTRES - QUARTIER LATIN INSTITUT NATIONAL DE LA SANTE ET DE LA RECHERCHE MEDICALE (INSERM) UNIVERSITE PARIS DESCARTES CENTRE NATIONAL DE LA RECHERCHE SCIENTIFIQUE (CNRS)
<120> MICROFLUIDIC DEVICE WITH ONE MICROCHANNEL FOR MULTIPLE DETECTION
<130> B368607D34013
<150> EP14306617.3
   <151> 2014-10-13
<160> 1
<170> PatentIn version 3.5
<210> 1
   <211> 75
   <212> DNA
   <213> Artificial Sequence
<220>
   <223> Aptamer
<220>
   <221> misc_binding
   <222> (1)..(1)
   <223> 6-carboxyfluorescein (6-FAM) at the 5' end
<220>
   <221> misc_binding
   <222> (75)..(75)
   <223> 5-Octadiynyl at the 3' end
<400> 1
   ataccagctt attcaattgc aacgtggcgg tcagtcagcg ggtggtgggt tcggtccaga        60
   tagtaagtgc aatct                                                          75

## Description

The present invention relates to a microfluidic device comprising at least a microchannel, the surface of which comprises at least distinct areas each grafted with a ligand, as well as a method for manufacturing such a microfluidic device and its use for the detection of targets capable of binding to the ligands.

After the conception of manual and then automatic analysis systems on a macroscopic level, microfluidic devices allow now the reduction of the volume of the consumables, the wastes, as well as the samples to be tested.

Such microfluidic devices are well adapted to detect and/or quantify the presence of one biological or chemical species (target) in a sample but there exists still a need for a microfluidic device which is easy to manufacture and which allows the detection and/or quantification of several targets present in a same sample in one step. WO 2010/019969 A1 discloses a microfluidic device with material that contains a target (see abstract).

The aim of the present invention is thus to provide a microfluidic device comprising at least a microchannel wherein it is possible to quantitatively detect in one step, the presence of several targets in a liquid sample, even at trace level.

The present invention relates thus to a microfluidic device comprising a support part and a cover part defining together at least a microchannel, and notably one microchannel, said microchannel having a surface, said surface comprising:
- a first area which is grafted with a first ligand, and
- at least a second area which is distinct from the first area and which is grafted with a second ligand which is different from the first ligand,
wherein each ligand is capable of binding to a target, the targets being different from each other.

The proposed device allows simultaneously extracting and concentrating different targets contained in one same sample on the different grafted areas while the sample is circulating in the microchannel. Once extracted and concentrated, the targets may be quantified by a detection device.

In addition, the proposed device allows analysing complex samples containing small quantities of targets, notably in the trace level. This may be particularly advantageous in case of dangerous samples, such as samples containing radioactive elements for instance, or samples containing element, molecules or ions for instance which may not be available in large quantities, such as biological samples or environmental samples for example.

The invention will be described by way of example, with reference to the accompanying drawings in which:
Figures 1A and 1B diagrammatically show a microfluidic device according to a possible embodiment of the invention,
Figure 2 diagrammatically shows a microfluidic detection system according to a possible embodiment of the invention,
Figure 3 diagrammatically illustrates steps of a method of manufacturing a microfluidic device according to a first embodiment of the invention,
Figures 4 to 8 diagrammatically illustrates steps of the method according to the first embodiment of the invention,
Figure 9 diagrammatically illustrates steps of the method according to a second embodiment of the invention.

### Microfluidic device:

By "microchannel" is meant in the present invention channel having a cross section which has dimensions in the micrometer range. Typically, the microchannel will have a width comprised between 10 and 1000 µm, notably between 50 and 300 µm and a depth between 10 and 400 µm, notably between 10 and 50 µm. However, the length of the microchannel can be in the centimeter or decimeter range.

By "area" is meant in the present invention, a surface of microchannel on which is grafted a ligand that allows the extraction and concentration of a defined target.

The microfluidic device according to the present invention comprises a support part and a cover part. Typically, the support part is engraved with a groove allowing the formation of the microchannel when the support part is covered with the cover part.

Typically, the microchannel has a rectangular cross section. In this case, the microchannel is constituted of four walls, i.e. one bottom wall, one top wall and two lateral walls. The bottom wall and the lateral walls are constituted by the walls of the groove, whereas the top wall is part of the surface of the cover part. Each of the grafted areas can be located on one or several of these walls. Typically, each of the grafted areas will be located on the bottom wall (i.e. on the support part) or on the top wall (i.e. on the cover part).

The support and covert parts can be made in any material. Typically, the support and cover part will be made in material conventionally used for microfluidic devices. For example, the support and covert parts can be made in a conductive or semiconductive material, silicon, glass or a polymer material. The support and cover parts can be made in different materials.

Preferably, the support part and/or the cover part (and more particularly the part(s) bearing the grafted area) is/are made in a polymer material. The polymer material will be advantageously a cyclic olefin copolymer (COC) such as a copolymer of ethylene and norbornene or tetracyclododecene; a cyclic olefin polymer (COP); or a fluorinated polymer such as a terpolymer of tetrafluoroethylene (F₂C=CF₂), hexafluoropropylene (F₂C=CF-CF₃) and vinylidene (H₂C=CF₂) (Dyneon™ THV).

In particular, the surface of the microchannel can comprise N distinct areas with N being equal or above 2 and notably being equal or below 10, in particular being equal or below 5, each area being grafted with a ligand, the N ligands being different from each other and each ligand being capable of binding to a target, the N targets being different from each other.

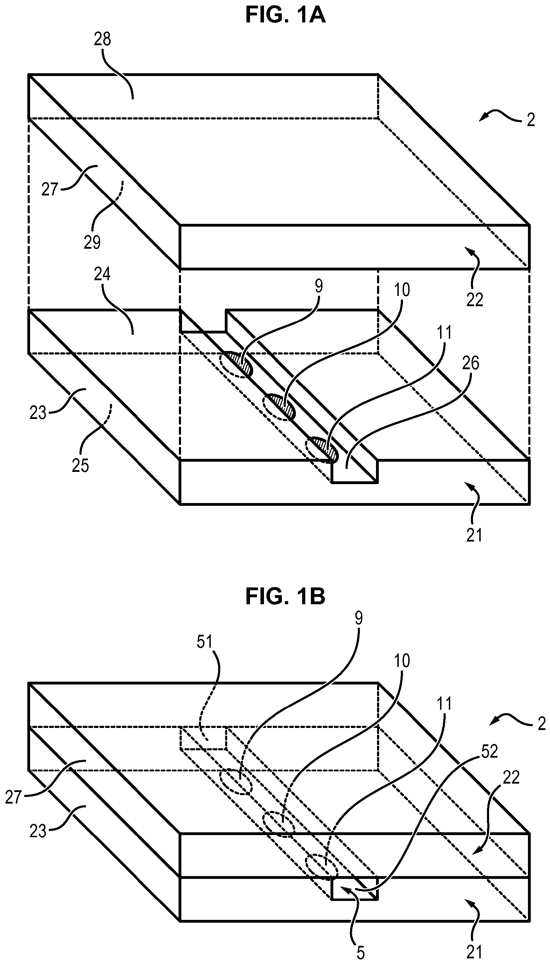

Figures 1A and 1B illustrate a microfluidic device 2 according to a possible embodiment of the invention.

In the example illustrated on these figures, the device 2 comprises a support part 21 and a cover part 22. The support part 21 comprises a layer 23 of material (for instance a fluorinated material) having an upper face 24 and a lower face 25. The upper face 24 has been etched so as to form a groove 26 in the layer 23 of material.

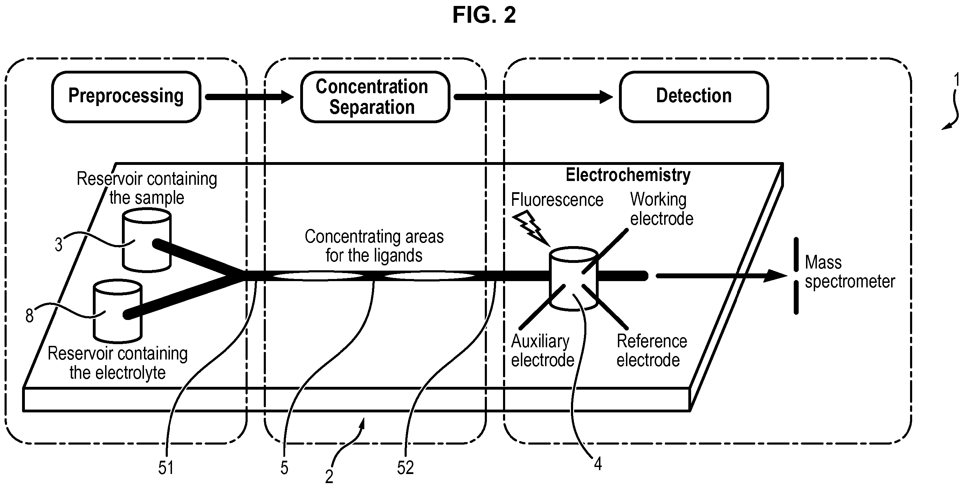

The cover part 22 may comprise a layer of material 27 (for instance glass) having an upper face 28 and a lower face 29. The cover part 22 is intended to be mounted on the support part 21 as shown on figure 2B, so as to close the groove 26. More precisely, the cover part 22 is positioned with its lower face 29 in contact with the upper face 24 of the support part 21. The cover part 22 is sealed on the support part 21.

Once the cover part 22 is mounted on the support part 21 (Figure 1B), the groove defines a microchannel 5 extending between the support part 21 and the cover part 22. The surface of the microchannel 5 is defined by the inner surface of the groove 26 of the support part 21 and the lower face 29 of the cover part 22, extending over the groove 26.

The microchannel 5 has an input 51 and an output 52. A sample to be analysed may be circulated through the microchannel from the input 51 to the output 52.

The surface of the microchannel 5 comprises several areas 9 to 11 which are grafted with respective ligands. The ligands are different from one area to the others. In particular, each ligand is capable of binding to a specific target, the targets being different from each others. As a result, each individual area 9 to 11 is capable of extracting and concentrating a respective target contained in the sample while the sample is circulated through the microchannel 5.

According to a first embodiment, the support and/or the cover part(s) bearing the grafted areas is/are made in a fluorinated material, in particular a fluorinated polymer such as a terpolymer of tetrafluoroethylene (F₂C=CF₂), hexafluoropropylene (F₂C=CF-CF₃) and vinylidene (H₂C=CF₂) (Dyneon™ THV), and the ligands are grafted to the areas of the microchannel by means of a linker, such as a benzene-(C₀-Cₙ)alkyl-1,2,3-triazole group, in particular a benzene-(C₀-C₆)alkyl-1,2,3-triazole group (divalent group), the benzene moiety being linked to the surface of the microchannel and the 1,2,3-triazole moiety being linked to the ligand.

By "(C₀-Cₙ)alkyl" is meant in the present invention a single bond or a (C₁-Cn)alkyl group.

By "(C₀-C₆)alkyl" is meant in the present invention a single bond or a (C₁-C₆)alkyl group.

By "(C₁-Cₙ)alkyl" is meant in the present invention a straight or branched saturated hydrocarbon chain containing from 1 to n carbon atoms with n being an integer above 2 including, but not limited to, methyl, ethyl, n-propyl, iso-propyl, n-butyl, iso-butyl, sec-butyl, t-butyl, n-pentyl, n-hexyl, and the like.

By "(C₁-C₆)alkyl" is meant in the present invention a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

According to a second embodiment, the ligands are grafted to the areas of the microchannel by means of a linker, such as a 1,2,3-triazole group.

In this case, the support and/or the cover part(s) bearing the grafted areas is/are made in any material, and in particular in a polymer material such as a cyclic olefin copolymer (COC) such as a copolymer of ethylene and norbornene or tetracyclododecene; a cyclic olefin polymer (COP); or a fluorinated polymer such as a terpolymer of tetrafluoroethylene (F₂C=CF₂), hexafluoropropylene (F₂C=CF-CF₃) and vinylidene (H₂C=CF₂) (Dyneon™ THV).

### Ligands and targets:

By "ligand" is meant in the present invention an entity, in particular a chemical or biological entity, capable of selectively binding to a defined target.

The ligand can be in particular, but non limiting, aptamers, antibodies, nanobodies, immunoglobulins, enzymes, receptors, chelatants, biomimetic molecules, etc.

By "target" is meant in the present invention an entity or a family of closely related entities which can be bound to a ligand. Indeed, a ligand can be capable of binding to only one entity (the binding is thus selective and specific) or to a family of closely related entities having close structures (the binding is only selective in this case).

The target can be a chemical or biological entity or family of entities such as an inorganic ion, a small molecule, a cell, a virus, etc.

By "bind", "binding", "bound" is meant in the present invention that the target is caught / trapped by the ligand by means of any interaction, such as, but non limiting, electrostatic interaction, van der Waals interaction, inclusion phenomena.

### Method for manufacturing the microfluidic device:

The present invention relates also to a method for manufacturing a micro fluidic device according to the invention, comprising the following successive steps:
(1) providing a micro fluidic device comprising a support part and a cover part defining together a microchannel,
(2) grafting a first ligand in a first area of the surface of the microchannel, and
(3) grafting at least a second ligand, which is different from the first ligand, in at least a second area of the surface of the microchannel.

If the microchannel comprises N areas grafted with a ligand as defined previously, the grafting step (step (2) or (3)) is reiterated N times successively.

The methods for providing a micro fluidic device comprising a support part and a cover part defining together a microchannel are well known to the one skilled in the art. For providing such a microfluidic device, it is necessary notably to provide a support part engraved with a groove, as well as a cover part. Various methods exist to manufacture such a support engraved with a groove. Notably, it is possible to engrave the groove directly on the solid support. However, when the support is made in a polymer material, it is possible to mould the support by pressing the polymer material on a mould comprising the pattern of the groove.

The grafting steps (2) and (3) will be performed on the support part (in the groove) and/or the cover part (i.e. the part(s) bearing the areas of the microchannel to be grafted). Once the areas are grafted, the cover part is mounted on the support part.

Various methods can be used to graft several distinct areas with distinct ligands (grafting steps (2) and (3)). All these methods need to be able to lead to a ligand grafted in a very localized area and not on all the surface of the microchannel or on a large area of the surface of the microchannel (such as one or several of the walls of the microchannel).

The ligands can be grafted on an area of the surface of the microchannel by Click chemistry, and more particularly by a reaction between an azide function (-N₃) and an alkyne function (preferably a terminal alkyne function -C≡CH), also called azide-alkyne Huisgen cycloaddition. For that, the ligand is functionalized with an azide or alkyne function, whereas the area to be grafted is functionalized with the other function, i.e. respectively an alkyne or azide function. The azide and alkyne functions react together to form a 1,2,3-triazole by a 1,3-dipolar cycloaddition. Such a reaction is illustrated on the scheme below in the case where the azide function is present on the surface of the microchannel whereas the ligand is functionalised with an alkyne function.

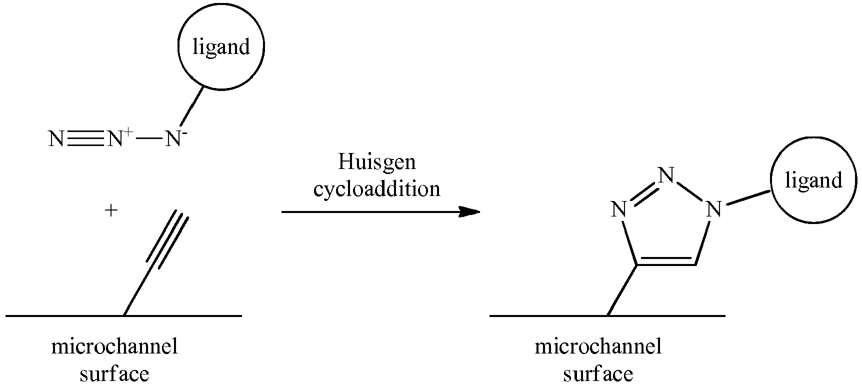

Such a cycloaddition reaction between an azide and an alkyne can be catalysed by a copper (I) catalyst such as CuBr or Cul. However, the copper (I) catalyst can be formed *in situ* by reduction of a copper (II) species, in particular by reduction of a copper (II) salt such as CuSO₄ in the presence of a reducing agent such as ascorbic acid or a salt thereof.

The cycloaddition can be performed in various solvents, such as alcohols (such as tert-butanol), dimethylsulfoxyde (DMSO), N,N-dimethylformamide (DMF), acetone, water or mixtures thereof.

In order to obtain a ligand grafted in a very localized area, two strategies are possible in the case of the use of Click chemistry for grafting the ligand on the surface of the microchannel:
(a) the functionalization of the microchannel surface with azide or alkyne functions is performed in a localized manner, i.e. only on a well-defined area of the microchannel is functionalised, and is followed by the grafting of the ligand by means of Click chemistry on the surface of the microchannel, which can be thus performed only in the area bearing the azide or alkyne functions, or
(b) the whole surface of the microchannel or at least a large part (for example the surface present only on the support part or on the cover part) of the microchannel is functionalised with azide or alkyne functions, and is followed by the grafting of the ligand performed in a localized manner, i.e. only on a well-defined area of the microchannel.

### Strategy (a):

When the surface of the microchannel to be grafted is made in a fluorinated material, such as a fluorinated polymer (for ex. a terpolymer of tetrafluoroethylene (F₂C=CF₂), hexafluoropropylene (F₂C=CF-CF₃) and vinylidene (H₂C=CF₂) (Dyneon™ THV)), this surface can be locally functionalised with azide (-N₃) or alkyne (-C≡CH) functions by carbonization of the area of the microchannel to be grafted to produce a carbonaceous area, followed by a reaction of the carbonaceous area with a benzene diazonium salt bearing an azide or alkyne function

Consequently, when the support and/or cover part bearing the area to be grafted is made in a fluorinated material, such as a fluorinated polymer (for ex. a terpolymer of tetrafluoroethylene (F₂C=CF₂), hexafluoropropylene (F₂C=CF-CF₃) and vinylidene (H₂C=CF₂) (Dyneon™ THV)), the grafting steps (2) and (3) can comprise the following steps:
(i) carbonizating the area of the microchannel to produce a carbonaceous area,
(ii) reacting the carbonaceous area with a benzene diazonium salt bearing an azide or alkyne function to give an area grafted with azide or alkyne functions, and
(iii) reacting the area grafted with azide or alkyne functions with a ligand bearing respectively an alkyne or azide function to obtain the area grafted with the ligand.

The localized carbonization step (i) can be assisted by scanning electrochemical microscopy (SECM) in the presence of a species capable of generating a radical anion, such as 2,2'-bipyridine, 4-phenylpyridine, benzonitrile or naphthalene, in particular such as 2,2'-bipyridine. Indeed, such a method allows the reduction of species capable of generating a radical anion only around the SECM electrode tip to generate locally radical anions leading to carbonization of the surface in a localised manner.

The size of the carbonaceous area will depend on the size and design of the electrode tip, on the distance of the electrode from the surface and on the speed of the electrode. The carbonaceous area can have various patterns by moving the electrode above the surface of the microchannel to be carbonized.

The SECM electrode can be an electrode made in conductive material such as platinum, carbon, gold, etc. According to a particular embodiment, the SECM electrode is in platinum. The diameter of the SECM electrode can be comprised between 1 and 50 µm, notably between 5 and 20 µm. The potential applied to the SECM electrode can be comprised between -2 and -2.5 V vs Ag/AgCl.

The benzene diazonium salt bearing an azide or alkyne function can then react with the carbonaceous area by auto-grafting in order to functionalize the area with azide or alkyne functions (step (ii)).

The benzene diazonium salt bearing an azide or alkyne function can be a salt of 4-azido-(C₀-Cₙ)alkyl-benzene diazonium or 4-acetylene-(C₀-Cₙ)alkyl-benzene diazonium, in particular of 4-azido-(C₀-C₆)alkyl-benzene diazonium or 4-acetylene-(C₀-C₆)alkyl-benzene diazonium. The salt can be in particular a chloride or a tetrafluoroborate.

This step (ii) can be performed in various solvents such as acetonitrile.

Step (iii) can be performed by Click chemistry as defined previously.

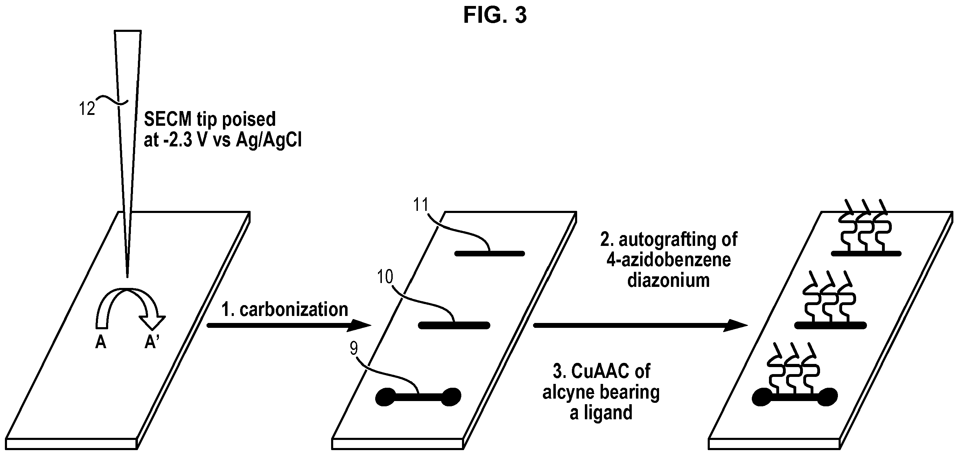

Figures 3 to 8 illustrate steps of the method for manufacturing a microfluidic device 2 according to strategy (a).

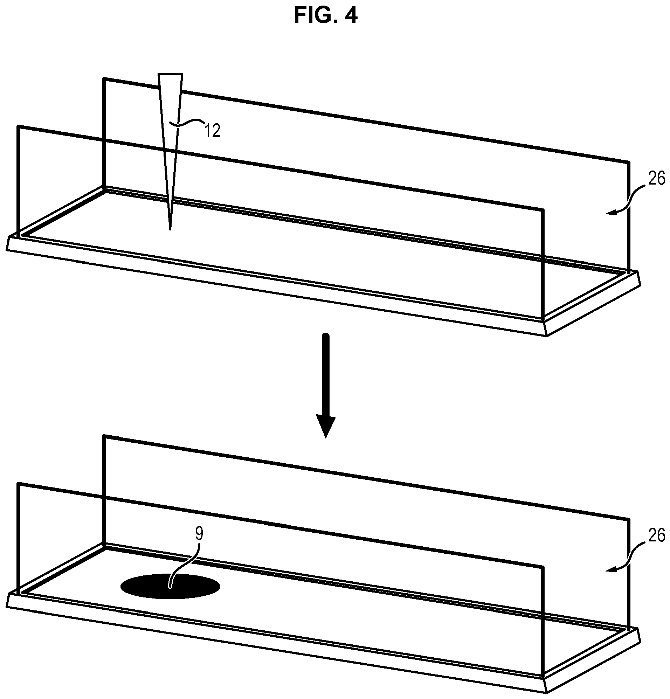

Figures 3 and 4 illustrate a step of carbonization of a first area 9 of the groove 26 by scanning the first area 9 with an electrode tip 12 so as to produce a first carbonaceous area 9.

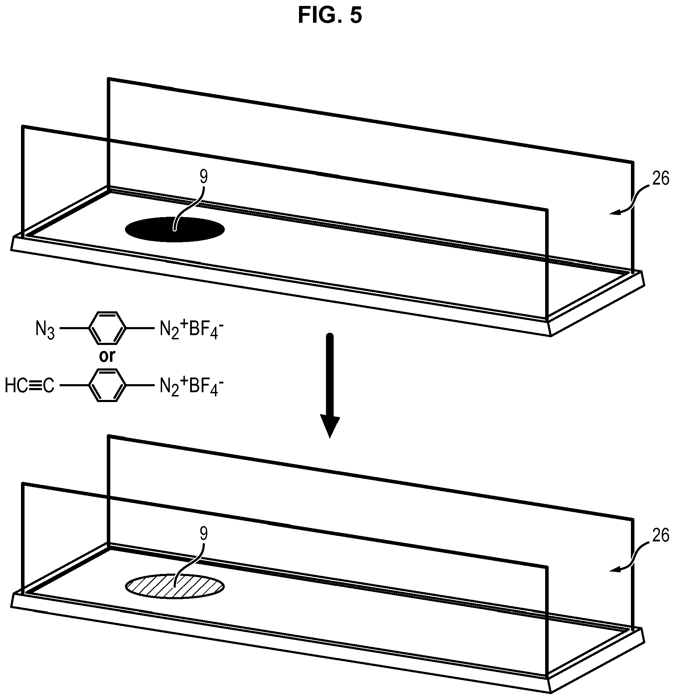

Figures 3 and 5 illustrate a step of reaction of the first carbonaceous area 9 with a benzene diazonium salt bearing an azide or alkyne function.

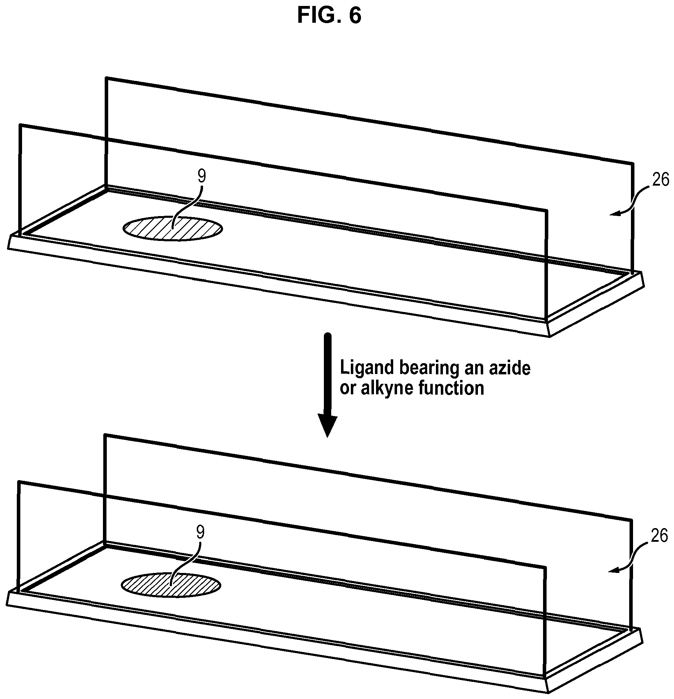

Figures 3 and 6 illustrate a step of reacting the first grafted area 9 with a first ligand bearing respectively an alkyne or azide function to obtain the first area 9 grafted with the first ligand.

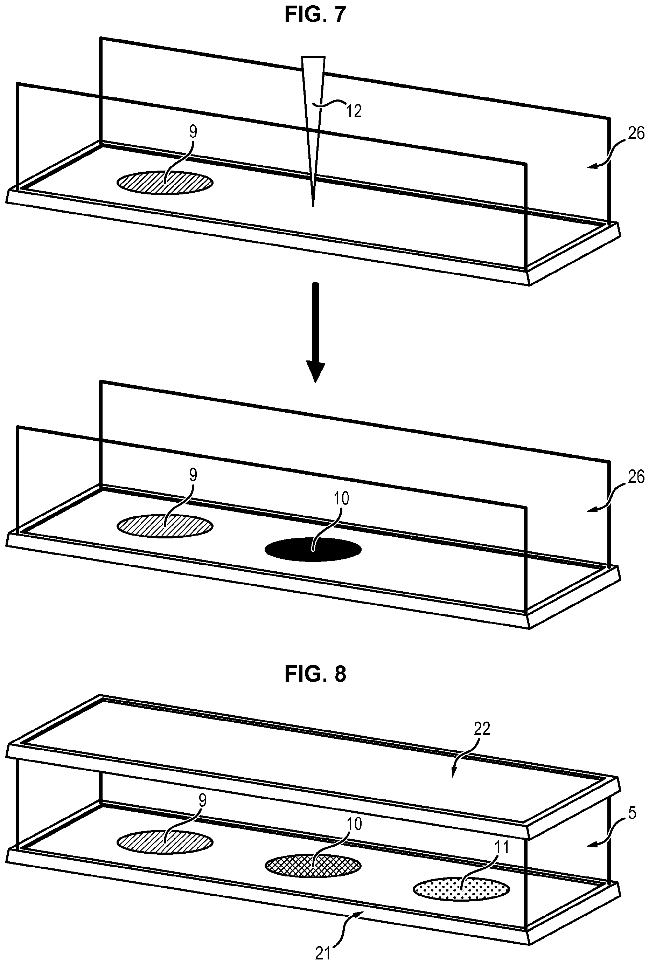

Figure 7 illustrates a step of carbonization of a second area 10 of the groove 26.

Figure 8 illustrates a step of closing the groove 26 by mounting the cover part 22 on the support part 21 so as to form the microchannel 5. As an example, the surface of the microchannel 5 comprises three areas 9, 10, 11 grafted respectively with three different ligands.

### Strategy (b):

A second strategy involves the functionalization of the whole surface of the microchannel or at least a large part (for example the surface present only on the support part or on the cover part) with azide or alkyne functions, followed by the grafting of the ligand performed in a localized manner.

In particular, the support and/or cover part(s) (more particularly the part(s) bearing the area to be grafted) will have a surface functionalized with azide or alkyne functions, notably with azide functions, and each of the grafting steps (2) and (3) can then be assisted by scanning electrochemical microscopy (SECM) in the presence of a ligand bearing respectively an azide or alkyne function, notably with alkyne functions, and a copper (II) salt such as CuSO₄.

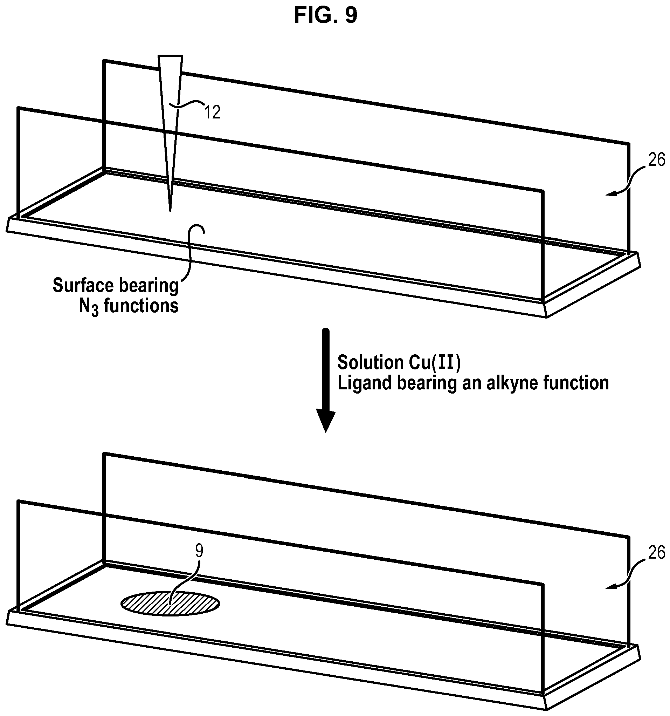

Figure 9 illustrates a step of grafting ligands in a first localized area 9 of the functionalized area.

Various methods can be used to functionalise the surface of the support and/or cover part(s) with azide or alkyne function.

A first method uses a plasma treatment. Such a treatment involves the polymerisation of a brominated monomer such as 1-bromopropane in a plasma reactor leading to the deposition of a thin brominated polymer layer on the surface of the support and/or cover part(s). Then, the obtained Br-modified support and/or cover part(s) is submitted to a chemical reaction to substitute the Br groups with N₃ functions via a nucleophilic substitution, notably in the presence of NaN₃ or an azido-(Co-Cₙ)alkylbenzene diazonium salt such as an azido-(C₀-C₆)alkylbenzene diazonium salt (for ex. chloride or tetrafluoroborate salt), to lead finally to azido-modified support and/or cover part(s).

A second method uses a photochemical treatment. Such a treatment involves an hydrogen atom abstraction from the surface of the support and/or cover part(s) in the presence of a photoinitiator (such as benzophenone) under UV irradiation (typically at 360 nm) in order to generate a radical anion which can then react with a brominated monomer (such as 1-bromopropane) or a brominated oligomer or polymer which could have been formed in the presence of the photoinitiator. This leads to the modification of the surface of the support and/or cover part(s) by a brominated coating. Then the obtained Br-modified support and/or cover part(s) is submitted to a chemical reaction to substitute the Br groups with N₃ functions via a nucleophilic substitution, notably in the presence of NaN₃ or an azido-(C₀-Cₙ)alkylbenzene diazonium salt such as an azido-(C₀-C₆)alkylbenzene diazonium salt (for ex. chloride or tetrafluoroborate salt), to lead finally to azido-modified support and/or cover part(s).

The grafting steps (2) and (3) are then performed by Click chemistry as defined previously but in a localised manner thanks to the use of SECM. Indeed, the SECM electrode allows reducing the copper (II) salt in a copper (I) species but only around the electrode tip. Now the cycloaddition of the azide with the alkyne can be performed only in the presence of a catalyst such as a copper (I) species (and not a copper (II) species). Consequently, the localized reduction of the copper (II) salt allows performing the Click chemistry in a localised manner.

The size of the grafted area will depend on the size and design of the electrode tip, on the distance of the electrode from the surface and on the speed of the electrode. The grafted area can have various patterns by moving the electrode above the surface of the microchannel to be grafted.

The SECM electrode can be an electrode in conductive material such as platinum, gold or carbon. According to a particular embodiment, the SECM electrode is in platinum. The diameter of the SECM electrode can be comprised between 1 and 50 µm, notably between 5 and 20 µm. The potential used can be comprised between -0.1 and -0.5 V vs Ag/AgCl.

### Microfluidic detection system:

The microfluidic device according to the invention can be part of a microfluidic detection system in order to allow the detection and quantification of the targets present in a sample to be analysed by a detection device.

The present invention relates also to a microfluidic detection system comprising:
- a microfluidic device according to the invention,
- a reservoir adapted for containing a sample to be analysed and connected to the inlet of the microchannel,
- a detection device for detecting the targets and connected to the outlet of the microchannel.

Figure 2 diagrammatically shows an example of a microfluidic detection system 1 according to an embodiment of the invention.

The microfluidic detection system 1 comprises thus, in addition to the microfluidic device 2, a reservoir 3 adapted for containing the sample to be analysed and a detection device 4 for detecting the targets.

The inlet 51 of the microchannel 5 of the microfluidic device 2 is connected to one or more reservoirs 3 containing the sample to be analysed and the outlet 52 of the microchannel 5 is connected to the detection device 4 for detecting the targets.

The microfluidic detection system 1 further comprises one or more reservoirs 8 containing electrolyte solutions and also connected to the inlet 51 of the microchannel 5.

Electrolyte solutions will be used notably for rinsing the microchannel 5 of the microfluidic device 2 and notably for releasing the targets from the ligands.

According to a variant, the reservoir for containing the electrolyte solution can be the same reservoir as the reservoir for containing the sample to be analysed. In this case, the content of the reservoir will be changed during the use of the microfluidic detection system depending on which solution is needed (i.e. the sample to be analysed or the electrolyte solution).

### Analysis method:

The present invention relates also to a method for analysing a sample containing targets using a microfluidic device according to the invention and more particularly a microfluidic detection system according to the invention, comprising:
(a) making the said sample, optionally dissolved in a solvent, circulating through the microchannel of the microfluidic device so as to allow the targets to bind to the ligands grafted on the areas of the microchannel,
(b) optionally releasing the targets from the ligands and migrating the released targets along the microchannel toward the detection device, and
(c) detecting each of the targets.

As illustrated on figure 1, each area 9, 10 of the microchannel grafted with a ligand is capable to bind to a defined target (corresponding to one entity or a family of closely related entities) allowing extracting selectively from the sample each of the targets and concentrating them in distinct areas in order to allow their quantitative detection.

Ligands can be very different from each other, so as to allow the extraction and concentration of no related various targets from a same sample, in a same microchannel.

Indeed, several areas of the microchannel can be grafted with various ligands available to bind to various defined targets not related (each corresponding to one entity or a family of closely related entities) allowing extracting selectively from the sample each of the targets and concentrating them in distinct areas in order to allow their quantitative detection.

Such an extraction and concentration step is performed by circulating the sample through the microchannel 5. The sample needs thus to be in a liquid form and can thus be dissolved beforehand in a solvent such as water, hydro-organic solvents or organic solvents. Thus, each of the targets is locally concentrated on the area of the microchannel by binding with the corresponding ligand. This step allows in particular to extract selectively and concentrate various targets from a sample which can be a very complex medium containing numerous chemical and biological entities.

Two options can be envisaged to detect and quantify the targets thus extracted and concentrated depending on the localisation of the detection device.

A first option is to place the detection device 4 along the microchannel 5. In this case, the detection can be typically performed by fluorescence, microscopy or electrochemistry. This first option is however not preferred since a detection by means of microscopy is not very sensitive and the detection by fluorescence implies that the ligand when it is not bound to the target and the ligand when it is bound to the target emit different fluorescent radiation, which is not always the case.

A second option is to place the detection device 4 at the outlet 52 of the microchannel 5. In this case, the detection device 4 can be performed for example by fluorescence, microscopy, electrochemistry or mass spectrometry.

In this case, the targets have first to be released from the ligands to which they were bound, in particular by a change of temperature, pH, ionic strength, medium, etc.

The released targets have then to be brought to the detection device 4. Such a migration step of the targets to the detection device can be performed for example by applying a pressure or an electric field. The advantage of the electric field is that the velocity of the targets will depend on their molecular weight and their charge and thus can be controlled electrokinetically. Thus, in this case, the velocity of the targets in the microchannel will be different from a target to another allowing a better separation of the targets from each other. In the case of a ligand capable of binding a family of related target entities, it will be possible also to separate the various target entities from each other. For obtaining a good separation of the various targets, it will be important to place the slowlier targets at the beginning of the microchannel (i.e. near the reservoir for the sample to be analysed) and the faster targets at the end of the microchannel (i.e. near the detection device).

This second option is thus preferred since it is more sensitive and more selective. Moreover, the migration of the targets under an electric field allows maintaining the targets (1) well separated to avoid that several targets reach the detection device at the same time and therefore to help for increasing selectivity; and (2) in a thin and focalized zone to lead to a more sensitive detection (for example, a thinner peak will be obtain on mass spectrum).

According to a preferred embodiment, an electrolyte solution can be first circulated in the microchannel 5 (for filing or rinsing the microchannel). Then the sample is circulated once or several times through the microchannel 5 to allow the various targets present in the sample to bind to the ligands and thus to be extracted and concentrated in localised areas of the microchannel. The same or another electrolyte solution is then circulated in the microchannel 5 to rinse the microchannel (due to the presence of possible impurities in the sample) and to release the targets from the ligands. An electric field is then applied in order to further separate the targets from each other if necessary and bring the targets in a separated way to the detection device 4 for their successive detection and quantification.

The circulation of the sample to be tested and the electrolyte solution can be carried out by means of a pressure system and/or an electric field.

Moreover, the electric field can be generated between a first electrode placed at the beginning of the microchannel and a second electrode place at the end of the microchannel.

The present invention is illustrated by the following non limitative examples.

### EXAMPLES

### Example 1 according to strategy (a):

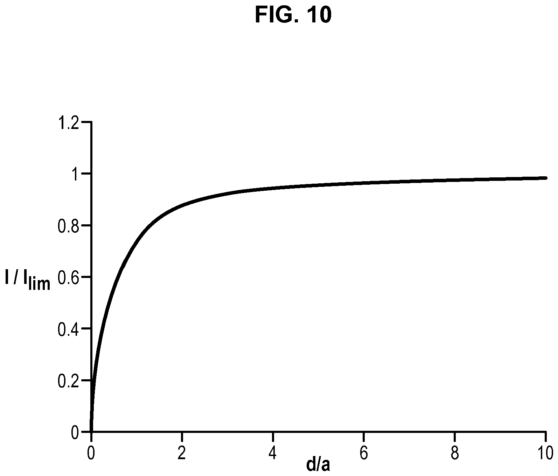

In a first step, a micrometric zone of a flat Dyneon® THV substrate was locally reduced and carbonized using a 25 µm diameter SECM tip (Pt ultramicroelectrode). The SECM tip was positioned in the vicinity of the surface and was used to locally reduce 2,2'-bipyridyl in DMF (dimethylformamide) solution to radical anion. Preliminary experiments confirmed that the reduction of 2,2'-bipyridyl starts at -2.2 V vs Ag/AgCl, and thus a potential of -2.3 V was used for the local patterning process. The tip was positioned at a desired close distance from the substrate using approach curve in feedback mode in a 0.1 M KCl + 5 mM ferrocene methanol aqueous solution which is represented on Figure 10. The tip electrode was stopped at a normalized distance value d from the substrate surface equal to 0.4 a, a being the SECM tip radius (in the micrometric range).

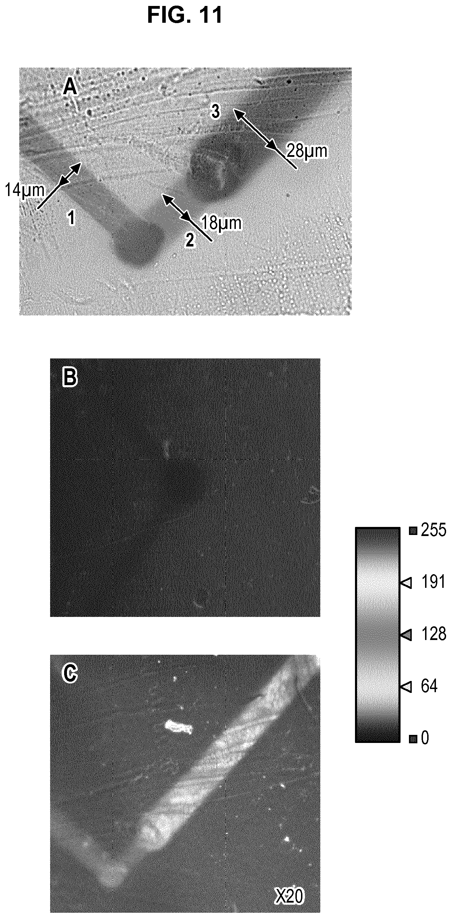

More precisely, to get the local carbonization, after rinsing the Dyneon® THV substrate with DMF, a solution of DMF containing 50 mM 2,2'-bipyridyl and 0.1 M Bu₄NBF₄ is introduced. The system was kept under nitrogen in a polyethylene bag (Aldrich) during the experiment. The humidity in the plastic bag was maintained at less than 30%, checked through a hair hygrometer. The tip was poised at -2.3 V to reduce 2,2'-bipyridyl while moving the electrode at scan rates of 1, 2 or 3 µm/s to create a micro localized carbonized areas on Dyneon® THV substrate. Figure 11A shows micrographs of Dyneon® THV substrate carbonization features: the carbonized areas appear as grey lines.

Then, the freshly carbonized surface was immersed in 5 mM 4-azidobenzenediazonium solution for 1 h to allow its spontaneous grafting, leading to the creation of terminal azide functional groups onto carbonized surfaces and thus of an N₃-modified Dyneon® THV substrate. In a final step, a fluorescent dye, acetylene-Fluor 488, was clicked through CuAAC reaction (Copper(I)-catalyzed Azide-Alkyne Cycloaddition). Figures 11B and 11C are optical microscope fluorescence images of the carbonized substrate grafted with the fluorescent dye showing that the immobilization of the fluorescent dye was carried out successfully and specifically on the carbonized areas. This also provides a visual means to evaluate CuAAC reaction yield on N₃-modified Dyneon® THV substrate following its carbonization.

In a second step, the same procedure was carried out within a microchannel of 950 µm width and 150 µm height engraved in the Dyneon® THV substrate. To do so, the SECM tip was placed on the edge of the microchannel at a normalized distance value d from the substrate surface equal to 0.4 *a* using conventional approach curve in feedback mode in a 0.1 M KCl + 5 mM ferrocene methanol aqueous solution, moved toward the micro channel center (about 50 µm away from the edge), and lifted down towards the micro channel bottom wall to a normalized distance d varying from 0.4 *a* to 0.15 *a*. The substrate was then immersed in a DMF solution containing 100 mM 2,2'-bipyridyl and 0.1 M Bu₄NBF₄. The carbonization reaction at the bottom surface of the microchannel through the electrochemical reduction of 2,2'-bipyridyl was performed at two different positions of the tip and at two different scan rates.

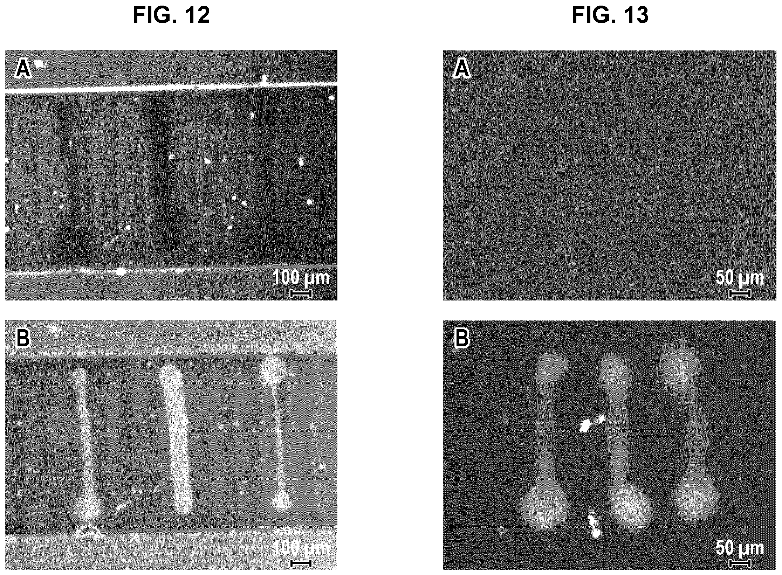

Figure 12 shows the optical microscope fluorescent image of the carbonized areas (A) after adsorption of 4-azidobenzenediazonium and (B) after the click reaction with the fluorescent dye acetylene-Fluor 488. Whatever the carbonization conditions used, these data allow confirming the successful specific micro immobilization of the fluorescent dye within the micro channel on the N₃-modified Dyneon® THV substrate following its carbonization. Pattern 1 was obtained by moving the tip at 3 µm/s when positioned at a normalized distance *d* = 0.4 *a*; Pattern 2 was obtained by moving the tip at 1 µm/s when positioned at a normalized distance *d* = 0.4 *a*; and Pattern 3 was obtained by moving the tip at 3 µm/s when positioned at a normalized distance *d* = 0.15 *a.*

As expected, for a given normalized distance, the decrease of the scan rate during the reduction of 2,2'-bipyridyl leads to larger carbonized patterns on Dyneon® THV substrate thus producing larger modified areas (≈ 28 µm wide at 1 µm/s and ≈ 14 µm wide at 3 µm/s) after click reaction with acetylene-Fluor 488. For a given scan rate during carbonization, lower working distance (between the tip SECM and the Dyneon® THV substrate) leads to narrower carbonized zone (≈ 65 µm at a normalized distance *d* = 0.4 *a* and ≈ 30 µm at a normalized distance *d* = 0.15 *a*) due to the convection induced by the tip movement on the reactant expansion.

Finally, the patterning of an aptamer of 70 bases with sequence 5'ATACCAGCTTATTCAATTGCAACGTGGCGGTCAGTCAGCGGGTGGTGGGT TCGGTCCAGATAGTAAGTGCAATCT-3' modified with 6-carboxyfluorescein (6-FAM) at the 5' end and 5-Octadiynyl at the 3' end was successfully performed following the same procedure.

Figure 13 shows an example of the obtained pattern drawn on the bottom wall of a microchannel with a tip of 10 µm diameter positioned at a normalized distance d = 0.4 of the substrate and moved at 1 µm/s. Figure 13A is thus an optical microscope fluorescence image of electro assisted carbonization of the engraved micro channel after immersion in 5 mM 4-azidobenzene diazonium solution and Figure 13B is an optical microscope fluorescence image after CuAAC reaction of the azide-functionalized patterned Dyneon® THV substrate with alkyne-modified aptamer. The three patterns were obtained by moving the tip at 1 µm/s when positioned at a normalized distance d = 0.4 a

### Example 2 according to strategy (b):

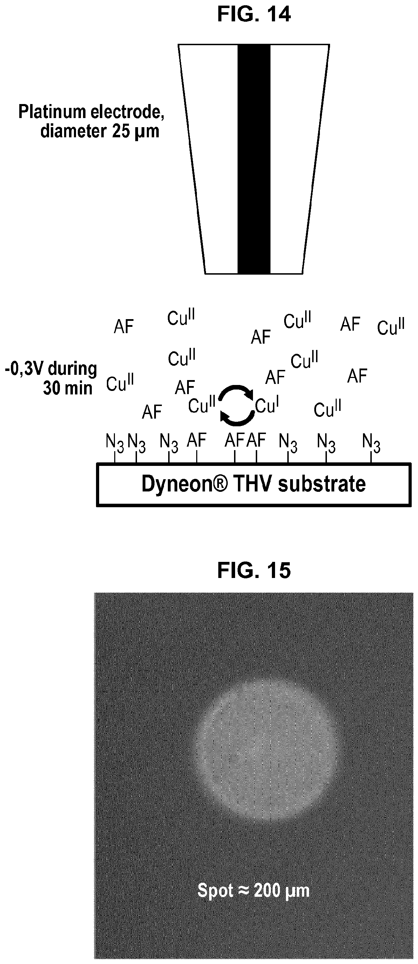

In this approach, the Dyneon® THV substrate was first functionalized by plasma processes in order to deposit a brominated polymeric layer. Then, bromide functions were replaced by azido functions using a classical nucleophilic substitution in NaN₃ solution. To this aim, the brominated Dyneon® THV substrate was immersed in a solution of EtOH/H₂O (1:1) containing NaN₃ (1M, pH 5, 5 % NaI) for 6 h at 50°C. The sample was then removed and washed with EtOH and ultra-pure water (≥ 18.2 MΩ). Then, the Dyneon® THV substrate was placed in the SECM cell and immersed in an aqueous solution containing Cu(II)SO4 and acetylene-Fluor 488. The SECM tip was positioned at d ≈10 µm above the surface of the azido-modified substrate, and Cu+ ions were produced electrochemically at the tip. This is aimed at locally triggering the CuAAC reaction between azido moieties present on the Dyneon® THV surface and alkyne functions present on the molecule to be immobilized (alkyne-modified ligand), here acetylene-Fluor (AF) 488, as shown on Figure 14. Preliminary experiments confirmed that a reduction process of Cu2+ starts at -0.1 V vs Ag/AgCl, and thus a potential of -0.3 V was used for the local click procedure. The tip was maintained for 30 minutes above the sample, leading to the modification of the surface in the shape of a "spot", as illustrated on Figure 15. Although this process probably corresponds to the reduction of Cu2+ to Cu0, a small amount of Cu+ is also present and this small amount can be enough to catalyze the click chemistry reaction.

## Claims

1. A micro fluidic device (2) comprising a support part (21) and a cover part (22) defining together a microchannel (5), said microchannel (5) having a surface, said surface comprising:
- a first area (9) which is grafted with a first ligand, and
- at least a second area (10) which is distinct from the first area (9) and which is grafted with a second ligand which is different from the first ligand,
wherein each ligand is capable of binding to a target, the targets being different from each other.

2. The micro fluidic device according to claim 1, wherein the surface of the microchannel comprises N distinct areas (9-11) with N being equal or above 2 and notably being equal or below 10, each area being grafted with a ligand, the N ligands being different from each other and each ligand being capable of binding to a target, the N targets being different from each other.

3. The microfluidic device according to any one of claims 1 and 2, wherein each ligand is chosen from among aptamers, antibodies, nanobodies, immunoglobulins, enzymes, receptors, chelatants and biomimetic molecules.

4. The micro fluidic device according to any one of claims 1 to 3, wherein the support (21) and/or the cover part(s) (22) bearing the grafted areas (9-11) is/are made in a polymer material, in particular a cyclic olefin copolymer (COC) such a copolymer of ethylene and norbornene or tetracyclododecene; a cyclic olefin polymer (COP); or a fluorinated polymer such as a terpolymer of tetrafluoroethylene (F₂C=CF₂), hexafluoropropylene (F₂C=CF-CF₃) and vinylidene (H₂C=CF₂).

5. The micro fluidic device according to any one of claims 1 to 4, wherein the support (21) and/or the cover part(s) (22) bearing the grafted areas (9-11) is/are made in a fluorinated material, such as a fluorinated polymer, and the ligands are grafted to the areas of the microchannel by means of a linker, such as a benzene-(C₀-C₆)alkyl-1,2,3-triazole group.

6. The microfluidic device according to any one of claims 1 to 4, wherein the ligands are grafted to the areas (9-11) of the microchannel (5) by means of a linker, such as a 1,2,3-triazole group.

7. The microfluidic device according to any one of claims 1 to 6, wherein the microchannel (5) comprises an inlet (51) and an outlet (52),
the inlet (51) being adapted to be connected to a reservoir (3) containing a sample to be analysed, and
the outlet (52) being adapted to be connected to a detection device (4) for detecting the targets.

8. A microfluidic detection system (1) comprising:
- a microfluidic device (2) according to claim 7,
- one or more reservoirs (3) adapted for containing a sample to be analysed and connected to the inlet (51) of the microchannel (5), and
- a detection device (4) for detecting the targets and connected to the outlet (52) of the microchannel (5).

9. A microfluidic detection system according to claim 8, further comprising one or more reservoirs (8) adapted for containing an electrolyte solution and connected to the inlet (51) of the microchannel (5).

10. A method for manufacturing a microfluidic device according to any one of claims 1 to 7, comprising the following successive steps:
(1) providing a microfluidic device (2) comprising a support part (21) and a cover part (22) defining together a microchannel (5),
(2) grafting a first ligand in a first area (9) of the surface of the microchannel (5), and
(3) grafting at least a second ligand, which is different from the first ligand, in at least a second area (10) of the surface of the microchannel (5).

11. The method according to claim 10, wherein the support (21) and/or the cover part(s) (22) bearing the grafted areas (9-11) is/are made in a fluorinated material, such as a fluorinated polymer, and each of the grafting steps (2) and (3) comprises the following steps:
(i) carbonizating the area of the microchannel (5) to produce a carbonaceous area (9),
(ii) reacting the carbonaceous area (9) with a benzene diazonium salt bearing an azide or alkyne function to give an area (9) grafted with azide or alkyne functions, and
(iii) reacting the area (9) grafted with azide or alkyne functions with a ligand bearing respectively an alkyne or azide function to obtain the area (9) grafted with the ligand.

12. The method according to claim 11, wherein the carbonization step (i) is assisted by scanning electrochemical microscopy (SECM) in the presence of a species capable of generating a radical anion such as 2,2'-bipyridine, 4-phenylpyridine, benzonitrile or naphthalene.

13. The method according to any one of claims 11 and 12, wherein the step (iii) is performed in the presence of a copper (I) catalyst, such as CuBr, CuI or a copper (I) catalyst prepared *in situ* by reduction of a copper (II) salt such as CuSO₄ in the presence of a reducing agent such as ascorbic acid or a salt thereof.

14. The method according to claim 10, wherein the support has a surface functionalized with azide functions and each of the grafting steps (2) and (3) is assisted by scanning electrochemical microscopy (SECM) in the presence of a ligand bearing an alkyne function and a copper (II) salt such as CuSO₄.

15. A method for analysing a sample containing targets using the microfluidic device (2) according to any one of claims 1 to 7 or the microfluidic detection system (1) according to claim 8 or 9 comprising:
(a) making the said sample, optionally dissolved in a solvent, circulating through the microchannel (5) of the microfluidic device (2) so as to allow the targets to bind to the ligands grafted on the areas (9-11) of the microchannel (5),
(b) optionally releasing the targets from the ligands and migrating the released targets along the microchannel (5) toward a detection device (4), and
(c) detecting and quantifying each of the targets.

## Patentansprüche

1. Mikrofluidikvorrichtung (2), umfassend ein Tragteil (21) und ein Abdeckteil (22), die gemeinsam einen Mikrokanal (5) definieren, wobei der Mikrokanal (5) eine Oberfläche aufweist, wobei die Oberfläche Folgendes umfasst:
- einen ersten Bereich (9), der mit einem ersten Liganden gepfropft ist, und
- zumindest einen zweiten Bereich (10), der sich vom ersten Bereich (9) unterscheidet, und der mit einem zweiten Liganden gepfropft ist, der sich vom ersten Liganden unterscheidet,
wobei jeder Ligand fähig ist, sich an ein Ziel zu binden, wobei sich die Ziele voneinander unterscheiden.

2. Mikrofluidikvorrichtung nach Anspruch 1, wobei die Oberfläche des Mikrokanals N verschiedene Bereiche (9-11) umfasst, wobei N gleich oder größer als 2 und insbesondere gleich oder kleiner als 10 ist, wobei jeder Bereich mit einem Liganden gepfropft ist, wobei die N Liganden voneinander unterscheiden und jeder Ligand fähig ist, sich an ein Ziel zu binden, wobei die N Ziele voneinander unterscheiden.

3. Mikrofluidikvorrichtung nach einem der Ansprüche 1 und 2, wobei jeder Ligand unter Aptameren, Antikörpern, Nanokörpern, Immunglobulinen, Enzymen, Rezeptoren, Chelatbildnern und biomimetischen Molekülen ausgewählt wird.

4. Mikrofluidikvorrichtung nach einem der Ansprüche 1 bis 3, wobei das Tragteil (21) und/oder das Abdeckteil (22), das/die die gepfropften Bereiche (9-11) trägt/tragen, aus einem Polymermaterial besteht/en, insbesondere einem Cyclo-Olefin-Copolymer (COC), wie einem Copolymer aus Ethylen und Norbornen oder Tetracyclododecen; einem Cyclic Olefin Polymer (COP); oder einem fluorierten Polymer, wie einem Terpolymer aus Tetrafluorethylen (F₂C=CF₂), Hexafluorpropylen (F₂C=CF-CF₃) und Vinyliden (H₂C=CF₂) .

5. Mikrofluidikvorrichtung nach einem der Ansprüche 1 bis 4, wobei das Tragteil (21) und/oder das Abdeckteil (22), das/die die gepfropften Bereiche (9-11) trägt/tragen, aus einem fluorierten Material, wie z. B. einem fluorierten Polymer, besteht/en und die Liganden vermittels eines Linkers, wie z. B. eine Benzol- (C₀-C₆)alkyl-1,2,3-Triazol-Gruppe, auf die Bereiche des Mikrokanals gepfropft sind.

6. Mikrofluidikvorrichtung nach einem der Ansprüche 1 bis 4, wobei die Liganden vermittels eines Linkers, wie einer 1,2,3-Triazol-Gruppe, auf die Bereiche (9-11) des Mikrokanals (5) gepfropft sind.

7. Mikrofluidikvorrichtung nach einem der Ansprüche 1 bis 6, wobei der Mikrokanal (5) einen Einlass (51) und einen Auslass (52) umfasst,
wobei der Einlass (51) so gestaltet ist, dass er mit einem Speicher (3) verbunden werden kann, der eine zu analysierende Probe enthält, und
wobei der Auslass (52) so gestaltet ist, dass er mit einer Erkennungsvorrichtung (4) zur Erkennung der Ziele verbunden werden kann.

8. Mikrofluidisches Erkennungssystem (1), umfassend:
- eine Mikrofluidikvorrichtung (2) nach Anspruch 7,
- einen oder mehrere Speicher (3), die zur Aufnahme einer zu analysierenden Probe gestaltet ist und mit dem Einlass (51) des Mikrokanals (5) verbunden sind, und
- eine Erkennungsvorrichtung (4) zur Erkennung der Ziele, die mit dem Auslass (52) des Mikrokanals (5) verbunden ist.

9. Mikrofluidisches Erkennungssystem nach Anspruch 8, das ferner einen oder mehrere Speicher (8) umfasst, die zur Aufnahme einer Elektrolytlösung gestaltet sind und mit dem Einlass (51) des Mikrokanals (5) verbunden sind.

10. Verfahren zur Herstellung einer Mikrofluidikvorrichtung nach einem der Ansprüche 1 bis 7, umfassend die folgenden aufeinanderfolgenden Schritte:
(1) Bereitstellen einer Mikrofluidikvorrichtung (2) umfassend ein Tragteil (21) und ein Abdeckteil (22), die gemeinsam einen Mikrokanal (5) definieren,
(2) Aufpfropfen eines ersten Liganden in einem ersten Bereich (9) der Oberfläche des Mikrokanals (5), und
(3) Aufpfropfen zumindest eines zweiten Liganden, der sich vom ersten Liganden unterscheidet, in zumindest einem zweiten Bereich (10) der Oberfläche des Mikrokanals (5).

11. Verfahren nach Anspruch 10, wobei das Tragteil (21) und/oder das Abdeckteil (22), das/die die gepfropften Bereiche (9-11) trägt/tragen, aus einem fluorierten Material, wie z. B. einem fluorierten Polymer, besteht/en und jeder Pfropfschritt (2) und (3) die folgenden Schritte umfasst:
(i) Karbonisieren des Bereichs des Mikrokanals (5), um einen kohlenstoffhaltigen Bereich (9) zu erzeugen,
(ii) Reagieren des kohlenstoffhaltigen Bereichs (9) mit einem Benzoldiazoniumsalz, das eine Azid- oder Alkinfunktion trägt, um einen mit Azid- oder Alkinfunktionen gepfropften Bereich (9) zu erhalten, und
(iii) Reagieren des mit Azid- oder Alkinfunktionen gepfropften Bereichs (9) mit einem Liganden, der eine Alkin- bzw. Azidfunktion trägt, um den mit dem Liganden gepfropften Bereich (9) zu erhalten.

12. Verfahren nach Anspruch 11, wobei der Karbonisierungsschritt (i) durch rasterelektrochemische Mikroskopie (RECM) in Gegenwart einer Spezies unterstützt wird, die fähig ist, ein Radikal-Anion wie 2,2'-Bipyridin, 4-Phenylpyridin, Benzonitril oder Naphthalin zu erzeugen.

13. Verfahren nach einem der Ansprüche 11 und 12, wobei der Schritt (iii) in Gegenwart eines Kupfer(I)-Katalysators, wie CuBr, CuI oder eines Kupfer(I)-Katalysators durchgeführt wird, der *in situ* durch Reduktion eines Kupfer(II)-Salzes wie CuSO₄ in Gegenwart eines Reduktionsmittels wie Ascorbinsäure oder eines Salzes davon hergestellt wird.

14. Verfahren nach Anspruch 10, wobei das Tragteil eine mit Azidfunktionen funktionalisierte Oberfläche aufweist und jeder der Pfropfschritte (2) und (3) durch rasterelektrochemische Mikroskopie (RECM) in Gegenwart eines Liganden mit Alkinfunktion und eines Kupfer(II)-Salzes wie CuSO₄ unterstützt wird.

15. Verfahren zum Analysieren einer Ziele enthaltenden Probe unter Verwendung der Mikrofluidikvorrichtung (2) nach einem der Ansprüche 1 bis 7 oder des mikrofluidischen Erkennungssystems (1) nach Anspruch 8 oder 9, umfassend:
(a) die optional in einem Lösungsmittel aufgelöste Probe durch den Mikrokanal (5) der Mikrofluidikvorrichtung (2) umlaufen lassen, damit sich die Ziele an die Liganden binden können, die auf die Bereiche (9-11) des Mikrokanals (5) aufgepfropft sind,
(b) optional die Ziele von den Liganden freisetzen und die freigesetzten Ziele entlang des Mikrokanals (5) zu einer Erkennungsvorrichtung (4) migrieren lassen, und
(c) jedes Ziel erkennen und quantifizieren.

## Revendications

1. Dispositif microfluidique (2) comprenant une partie support (21) et une partie couvercle (22) définissant ensemble un microcanal (5), ledit microcanal (5) ayant une surface, ladite surface comprenant :
- une première zone (9) qui est greffée avec un premier ligand, et
- au moins une deuxième zone (10) qui est distincte de la première zone (9) et qui est greffée avec un deuxième ligand qui est différent du premier ligand,
dans lequel chaque ligand est capable de se lier à une cible, les cibles étant mutuellement différentes.

2. Dispositif microfluidique selon la revendication 1, dans lequel la surface du microcanal comprend N zones distinctes (9-11), où N est égal ou supérieur à 2 et notamment égal ou inférieur à 10, chaque zone étant greffée avec un ligand, les N ligands étant mutuellement différents, et chaque ligand étant capable de se lier à une cible, les N cibles étant mutuellement différentes.

3. Dispositif microfluidique selon l'une quelconque des revendications 1 et 2, dans lequel chaque ligand est choisi parmi les aptamères, les anticorps, les nanocorps, les immunoglobulines, les enzymes, les récepteurs, les chélatants, et les molécules biomimétiques.

4. Dispositif microfluidique selon l'une quelconque des revendications 1 à 3, dans lequel la/les partie(s) support (21) et/ou couvercle (22) portant les zones greffées (9-11) est/sont faite(s) en un matériau polymère, en particulier un copolymère d'oléfine cyclique (COC) tel qu'un copolymère d'éthylène et de norbornène ou de tétracyclododécène ; un polymère d'oléfine cyclique (COP) ; ou un polymère fluoré tel qu'un terpolymère de tétrafluoroéthylène (F₂C=CF₂), d'hexafluoropropylène (F₂C=CF-CF₃) et de vinylidène (H₂C=CF₂) .

5. Dispositif microfluidique selon l'une quelconque des revendications 1 à 4, dans lequel la/les partie(s) support (21) et/ou couvercle (22) portant les zones greffées (9-11) est/sont faite(s) en un matériau fluoré, tel qu'un polymère fluoré, et les ligands sont greffés aux zones du microcanal au moyen d'un lieur, tel qu'un groupe benzène-(alkyle en C₀ à C₆)-1,2,3-triazole.

6. Dispositif microfluidique selon l'une quelconque des revendications 1 à 4, dans lequel les ligands sont greffés aux zones (9-11) du microcanal (5) au moyen d'un lieur, tel qu'un groupe 1,2,3-triazole.

7. Dispositif microfluidique selon l'une quelconque des revendications 1 à 6, dans lequel le microcanal (5) comprend une entrée (51) et une sortie (52),
l'entrée (51) étant adaptée pour être connectée à un réservoir (3) contenant un échantillon à analyser, et
la sortie (52) étant adaptée pour être connectée à un dispositif de détection (4) pour détecter les cibles.

8. Système de détection microfluidique (1) comprenant :
- un dispositif microfluidique (2) selon la revendication 7,
- un ou plusieurs réservoirs (3) adaptés pour contenir un échantillon à analyser et connectés à l'entrée (51) du microcanal (5), et
- un dispositif de détection (4) pour détecter les cibles et connecté à la sortie (52) du microcanal (5).

9. Système de détection microfluidique selon la revendication 8, comprenant en outre un ou plusieurs réservoirs (8) adaptés pour contenir une solution d'électrolyte et connectés à l'entrée (51) du microcanal (5) .

10. Procédé pour fabriquer un dispositif microfluidique selon l'une quelconque des revendications 1 à 7, comprenant les étapes successives suivantes :
(1) fourniture d'un dispositif microfluidique (2) comprenant une partie support (21) et une partie couvercle (22) définissant ensemble un microcanal (5),
(2) greffage d'un premier ligand dans une première zone (9) de la surface du microcanal (5), et
(3) greffage d'au moins un deuxième ligand, qui est différent du premier ligand, dans au moins une deuxième zone (10) de la surface du microcanal (5).

11. Procédé selon la revendication 10, dans lequel la/les parties support (21) et/ou couvercle (22) portant les zones greffées (9-11) est/sont faite(s) en un matériau fluoré, tel qu'un polymère fluoré, chacune des étapes de greffage (2) et (3) comprend les étapes suivantes :
(i) carbonisation de la zone du microcanal (5) pour produire une zone carbonée (9),
(ii) réaction de la zone carbonée (9) avec un sel de benzène-diazonium portant une fonction azoture ou alcyne pour donner une zone (9) greffée avec des fonctions azoture ou alcyne, et
(iii) réaction de la zone (9) greffée avec les fonctions azoture ou alcyne avec un ligand portant respectivement une fonction alcyne ou azoture pour obtenir la zone (9) greffée avec le ligand.

12. Procédé selon la revendication 11, dans lequel l'étape de carbonisation (i) est assistée par microscopie électrochimique à balayage (MECB) en présence d'une espèce capable de générer un anion radicalaire tel que 2,2'-bipyridine, 4-phénylpyridine, benzonitrile ou naphtalène.

13. Procédé selon l'une quelconque des revendications 11 et 12, dans lequel l'étape (iii) est effectuée en présence d'un catalyseur au cuivre(I), tel que CuBr, CuI ou un catalyseur au cuivre (I) préparé *in situ* par réduction d'un sel de cuivre(II) tel que CuSO₄ en présence d'un agent réducteur tel que l'acide ascorbique ou un sel de celui-ci.

14. Procédé selon la revendication 10, dans lequel le support a une surface fonctionnalisée avec des fonctions azoture et chacune des étapes de greffage (2) et (3) est assistée par microscopie électrochimique à balayage (MECB) en présence d'un ligand portant une fonction alcyne et d'un sel de cuivre(II) tel que CuSO₄.

15. Procédé pour analyser un échantillon contenant des cibles utilisant le dispositif microfluidique (2) selon l'une quelconque des revendications 1 à 7 ou le système de détection microfluidique (1) selon la revendication 8 ou 9, comprenant :
(a) la mise en circulation dudit échantillon, éventuellement dissous dans un solvant, dans le microcanal (5) du dispositif microfluidique (2) de façon à permettre aux cibles de se lier aux ligands greffés sur les zones (9-11) du microcanal (5),
(b) éventuellement la libération des cibles d'avec les ligands et la migration des cibles libérées le long du microcanal (5) en direction d'un dispositif de détection (4), et
(c) la détection et la quantification de chacune des cibles.
